# EUROPÄISCHE PATENTSCHRIFT

(11) **EP 2 281 533 B1**
(45) Veröffentlichungstag und Bekanntmachung des Hinweises auf die Patenterteilung: **05.06.2013**
(21) Anmeldenummer: 10008231.2
(22) Anmeldetag: 06.08.2010
(51) Int. Cl.: A61F 5/56

(54) **Antischnarchgerät**
Snore prevention device
Appareil anti-ronflement

(30) Priorität: 07.08.2009 DE 102009036510
(43) Veröffentlichungstag der Anmeldung: 09.02.2011
(73) Patentinhaber: Jochem, Ralf, 61348 Bad Homburg (DE)
(72) Erfinder: Husemann, Britta, 60439 Frankfurt am Main (DE)
(74) Vertreter: Jany und Petersen

(56) Entgegenhaltungen:
- US-A- 4 644 330
- US-A1- 2007 239 225
- US-B1- 6 270 466

## Beschreibung

Die Erfindung betrifft ein Antischnarchgerät mit einem Tragorgan, an dem ein Mikrofon und ein Signalprozessor zur Detektion von Schnarchgeräuschen, ein an ein Ohr anzusetzender Tongenerator zur Erzeugung eines akustischen Stimulationssignals und ein elektrischer Energiespeicher angebracht sind, wobei das Mikrofon und der Tongenerator durch einen einzigen elektroakustischen Wandler gebildet sind, das gemeinsame Tragorgan ein im Ohr zu tragendes Gehäuse ist und die Detektion nach einer bestimmten Zeitdauer selbsttätig ausschaltbar ist.

In der DE 44 06 860 ist ein Antischnarchgerät mit einem Tragorgan, an dem ein Mikrofon und ein Signalprozessor zur Detektion von Schnarchgeräuschen, ein an ein Ohr anzusetzender Tongenerator zur Erzeugung eines akustischen Stimulationssignals und ein elektrischer Energiespeicher angebracht sind, beschrieben. Es hat noch den Nachteil, dass seine Komponenten jeweils einzeln an einem Stirnband als Tragorgan befestigt sind, das Mikrofon z. B. in der Nähe des menschlichen Kehlkopfes, zwei Tongeneratoren in der Nähe der über Schallschläuche anzuschließenden Ohren und Signalprozessoren jeweils dazwischen. Das am Kopf anliegende Stirnband mit den daran befestigten Geräteteilen stört beim Einschlafen, insbesondere in den Seitenlagen des Kopfes.

Aus der US 6,270,466 B1 ist ein auf Biofeedback basierendes Antischnarchgerät bekannt, das ebenfalls ein Stirnband, ein Halsband, eine Brille oder eine ähnliche Einrichtung umfasst. Es kann für eine vorgegebene Zeitdauer eingeschaltet werden und kann sich bei Nichtbenutzung nach einer vorgegebenen Zeitdauer selbsttätig ausschalten.

In der US 2007/0239225 A1 ein Antischnarchgerät mit einem Tragorgan, an dem ein Mikrofon und ein Signalprozessor zur Detektion von Schnarchgeräuschen, ein an ein Ohr anzusetzender Tongenerator zur Erzeugung eines akustischen Stimulationssignls und ein elektrischer Energiespeicher angebracht sind, bekannt, wobei das Mikrofon und der Tongenerator durch einen einzigen elektroakustischen Wandler gebildet sind,

Aus der US 4,644,330 ist ein Antischnarchgerät mit einem Tragorgan, an dem ein Mikrofon und ein Signalprozessor zur Detektion von Schnarchgeräuschen, ein an ein Ohr anzusetzender Tongenerator zur Erzeugung eines akustischen Stimulationssignals und ein elektrischer Energiespeicher angebracht sind, bekannt, wobei das Mikrofon und der Tongenerator durch einen einzigen elektroakustischen Wandler gebildet sind, das gemeinsame Tragorgan ein im Ohr zu tragendes Gehäuse ist und die Detektion nach einer bestimmten Zeitdauer selbsttätig ausschaltbar ist. Das selbsttägige Ausschalten erfolgt, wenn während einer bestimmten Zeitdauer mehr als eine vorgegebene Anzahl von Schnarchgeräuschen detektiert wird.

Der Erfindung liegt die Aufgabe zugrunde, ein Antischnarchgerät der eingangs genannten Art zur Verfügung zu stellen, das von dem Benutzer leicht zu applizieren und bequem zu tragen und zu bedienen ist, so dass es ihn nur insoweit belästigt, wie es funktionsgemäß erforderlich ist.

Vorstehende Aufgabe wird bei Antischnarchgerät mit einem Tragorgan, an dem ein Mikrofon und ein Signalprozessor zur Detektion von Schnarchgeräuschen, ein an ein Ohr anzusetzender Tongenerator zur Erzeugung eines akustischen Stimulationssignals und ein elektrischer Energiespeicher angebracht sind, wobei das Mikrofon und der Tongenerator durch einen einzigen elektroakustischen Wandler gebildet sind, das gemeinsame Tragorgan ein im Ohr zu tragendes Gehäuse ist und die Detektion nach einer bestimmten Zeitdauer selbsttätig ausschaltbar ist, erfindungsgemäß dadurch gelöst, dass der Signalprozessor nur dann ein Stimulationssignal auslöst, wenn die Schallenergie im 10- bis 100-Hz-Prequenzbereich ein bestimmtes Ansprechniveau überschreitet und mindestens drei Schnarchgeräusche mit einem Intervall von 3 bis 8 Sekunden registriert wurden.

Der durch die Erfindung erzielte Vorteil der leichten Handhabung und besseren Trageigenschaft resultiert aus der Unterbringung aller notwendigen Bauteile in und an einem Gehäuse, das so klein ist, dass es in einem Ohr getragen werden kann. Allein mit der Miniaturisierung aller Bauteile ließe sich dieses Ziel jedoch nicht erreichen. Die Erfindung sieht deshalb als weitere Maßnahmen vor, dass das Mikrofon und der Tongenerator durch einen einzigen elektroakustischen Wandler gebildet sind, der darüber hinaus vorzugsweise auch noch als Anzeigeelement für die Einstellung der Lautstärke des Stimulationssignals, der Aufnahmeempfindlichkeit in der Mikrofonfunktion und des Ladezustands des elektrischen Energiespeichers dient.

Weiterhin ist vorgesehen, dass die Detektion nach einer bestimmten Zeitdauer von z. B. 2 bis 3 Stunden selbsttätig ausschaltbar ist. Auf diese Weise kommt man auch mit einem minimal kleinen Akkumulator als Energiespeicher aus. Die zeitliche Begrenzung der Detektion und Stimulation des Benutzers ist ohne weiteres möglich, weil der hauptsächliche Zweck des Antischnarchgeräts darin besteht, einer durch das Schnarchen gestörten Person, z. B. dem Ehepartner des Benutzers, Gelegenheit zum Einschlafen zu geben. Für den Benutzer hat das selbsttätige Ausschalten der Detektion nach 2 bis 3 Stunden den Vorteil, dass die REM-Phase seines Schlafs nicht oder jedenfalls weniger gestört wird als wenn die Detektion und Stimulation über die gesamte Nacht aktiviert bleiben.

Energie wird auch dadurch gespart, dass der Signalprozessor nicht schon jedes Mal nach einem Schnarchgeräusch ein Stimulationssignal auslöst. Es wird bevorzugt, ein Stimulationssignal erst dann zu erzeugen, wenn die Schallenergie im 10-bis 100-Hz-Frequenzbereich ein bestimmtes Ansprechniveau überschreitet und mindestens drei Schnarchgeräusche mit einem Intervall von 3 bis 8 Sekunden registriert wurden. Außerdem sollte das Intervall zwischen zwei Stimulationssignalen mindestens 15 Sekunden dauern.

In der bevorzugten praktischen Ausführung besteht das Gehäuse aus einem Unterteil und einem mit diesem verbundenen Deckel, wobei das Steuerorgan für die Lautstärke des Stimulationssignals ein im Gehäuse installiertes Potentiometer mit einem auf der Außenseite des Deckels angebrachten Stellknopf und das Steuerorgan für die Einstellung der Aufnahmeempfindlichkeit ein mit dem Signalprozessor zusammenwirkender, auf der Außenseite des Deckels zugänglicher Taster ist. Man kommt tatsächlich mit diesen zwei Steuerorganen aus, weil sie durch unterschiedliche Arten der Betätigung jeweils mehr als eine Funktion steuern können. So kann z. B. der Stellknopf des Potentiometers auch als Ein- oder Ausschalter für das Gerät dienen, und der Taster für die Einstellung der Aufnahmeempfindlichkeit kann durch unterschiedlich lange Betätigung auch zu seiner Umschaltung auf die akustische Anzeige dienen.

Der unterschiedlichen Form und Größe der Gehörgänge kann durch eine individuelle Anpassung des Gehäuseunterteils Rechnung getragen werden. Sie ist allerdings kostspielig. Um zu einer Produktion in größerer Serie zu kommen, ist deshalb auch eine Ausführung vorgesehen, bei der das den Tonein- und -ausgang des elektroakustischen Wandlers bildende untere Ende des Gehäuses mit einem Anschluss für unterschiedlich große Paßstücke versehen ist, die den Tonein- und -ausgang verlängern.

Ein Ausführungsbeispiel der Erfindung wird nachstehend anhand der Zeichnung näher erläutert. Es zeigen:
- Fig. 1: eine Seitenansicht des neuen Anti- schnarchgeräts;
- Fig. 2: eine um 90° gedrehte Seitenansicht des Antischnarchgeräts nach Fig. 1;
- Fig. 3: eine Draufsicht des Antischnarchgeräts nach Fig. 1 und 2;
- Fig. 4: einen Längsschnitt durch das Anti- schnarchgerät nach Fig. 1 bis 3;
- Fig. 5: eine perspektivische Ansicht der Unter- seite des Deckels des Antischnarchge- räts mit daran befestigten Bauteilen;
- Fig. 6: eine weitere perspektivische Ansicht mit anderer Blickrichtung des Gerätede- ckels nach Fig. 5;
- Fig. 7: ein Blockschaltbild der Ein- und Aus- gänge eines im Gerätegehäuse enthalte- nen digitalen Signalprozessors;
- Fig. 8: ein Schaubild der Funktionen der obers- ten Ebene, sowie
- Fig. 9: ein Schaubild der Benutzerschnittstel- le.

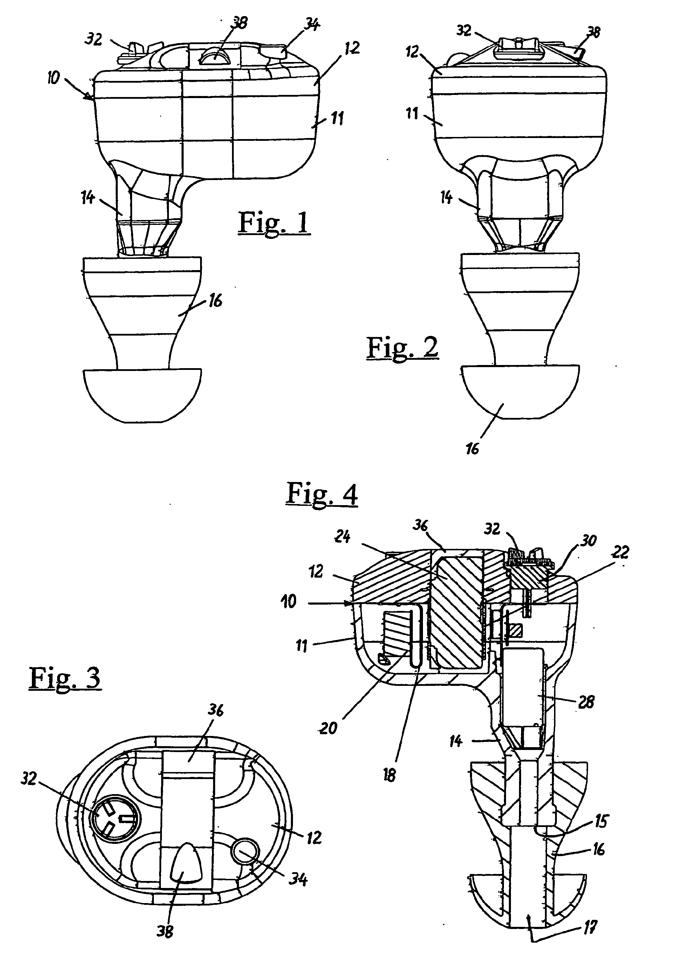

Das in Fig. 1 bis 4 in verschiedenen Ansichten gezeigte Antischnarchgerät hat ein Gehäuse 10, das im Ohr zu tragen ist und ein Gehäuseunterteil 11 sowie einen Deckel 12 aufweist. Das Gehäuseunterteil 11 hat die Form einer ovalen Schale, die im äußeren Ohr Platz findet. An einem Ende schließt sich nach unten eine einstückig angeformte, vor dem unteren Ende verjüngte, rohrförmige Verlängerung 14 an, die teilweise in den Gehörgang eingeführt werden kann, wobei der verjüngte Teil einen Tonein- und -ausgang bildet. An seinem unteren Ende befindet sich eine Anschlussöffnung 15, an deren Rand unterschiedlich große Paßstücke 16 angeschlossen werden können, die den Tonein- und -ausgang bis zu einer Schallaustrittsöffnung 17 verlängern und dazu dienen, das Gerät im Ohr zu halten. In einer teureren, individuellen Ausführung wird das Gehäuseunterteil 11, 14 entsprechend einem vom Ohr genommenen Abdruck geformt.

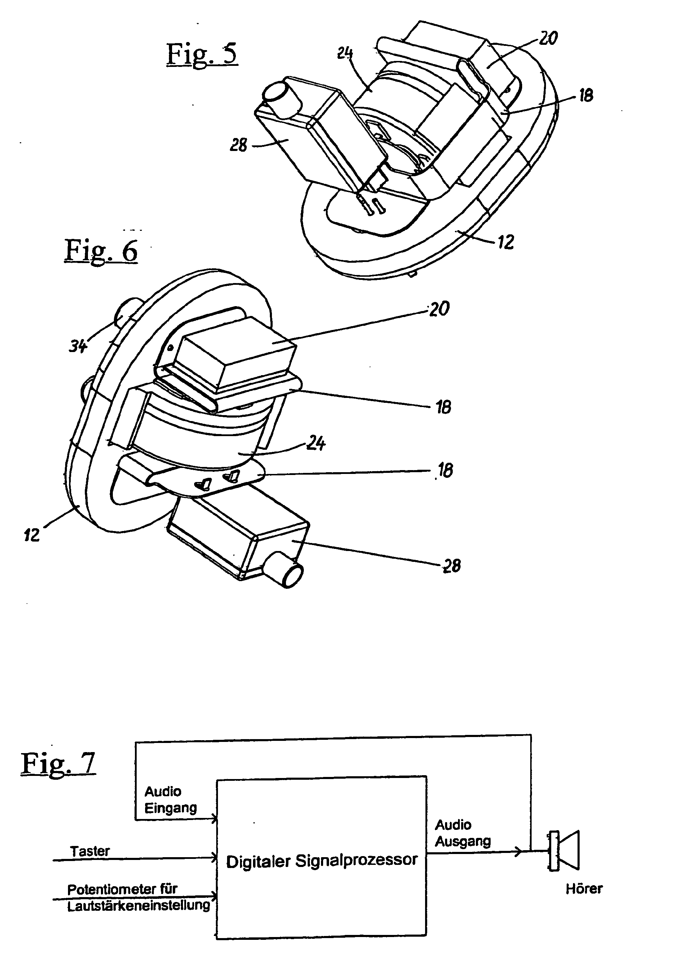

Wie am besten aus Fig. 5 und 6 hervorgeht, sind mehrere Bauteile des Antischnarchgeräts an der Unterseite des Deckels 12 montiert. Dies gilt insbesondere für eine gedruckte Leiterplatte 18 und einen mit dieser verbundenen, digitalen Signalprozessor 20. Außerdem sind an der Unterseite des Deckels 12 Kontakte 22 für eine Batterie 24 oder einen Akkumulator angebracht. Darüber hinaus ist an der mit dem Deckel 12 verbundenen Leiterplatte 18 ein im Gehäuseunterteil eingesetzter elektroakustischer Wandler bzw. Signal- und Schallgeber 28 angeschlossen, dessen Tonein- und -ausgang zur Schallaustrittsöffnung 17 hin weist und über letztere Schnarchgeräusche empfängt und akustische Stimulationssignale abgibt. Als digitaler Signalprozessor 20 kann ein Hybrid, z. B. Typ GA3280 von der Firma Sound Design Technology, Burlington/Ontario, Kanada dienen und als elektroakustischer Wandler z. B. der Typ RBB-04, 400 ohm von der Firma Star Micronics in Shizuoka, Japan.

Schließlich ist im Deckel 12 ein Potentiometer 30 mit einem von außen zugänglichen, drehbaren Stellknopf 32 als Steuerorgan gelagert. Ein ebenfalls von außen zugänglicher Taster 34 ist am Deckel 12 in der Nähe des digitalen Signalprozessors installiert und dient als Programmschalter. Um die Batterie bzw. den Akkumulator 24 entnehmen zu können, ohne den z. B. verklebten Deckel 12 öffnen zu müssen, ist dieser mit einem Batteriefach ausgebildet, das von einer leicht zu öffnenden Verschlussklappe 36 überdeckt ist. Das Ergreifen der Verschlussklappe 36 wird durch einen kleinen Griff 38 erleichtert.

Das Zusammenwirken der vorstehend genannten Bauteile ist in dem Blockschaltbild der Fig. 7 dargestellt. Danach hat der digitale Signalprozessor 20 einen Audioeingang für die vom elektroakustischen Wandler 28 bei der Schnarchdetektion erzeugten Signale, einen weiteren Eingang für die mittels des Tasters 34 eingegebenen Steuersignale und einen dritten Eingang für die am Potentiometer 30 vorgenommene Lautstärkeeinstellung für das akustische Stimulationssignal. Ein Audioausgang des digitalen Signalprozessors 20 ist mit dem elektroakustischen Wandler 28 verbunden. Darüber laufen Steuersignale zur Erzeugung von akustischen Stimulationssignalen und Anzeigesignalen betreffend den Ladezustand der Batterie 24, die Lautstärke des Stimulationssignals, die eingestellte Empfindlichkeit in der Mikrofonfunktion des elektroakustischen Wandlers 28 und die Bestätigung der Einschaltung der Schnarchdetektion.

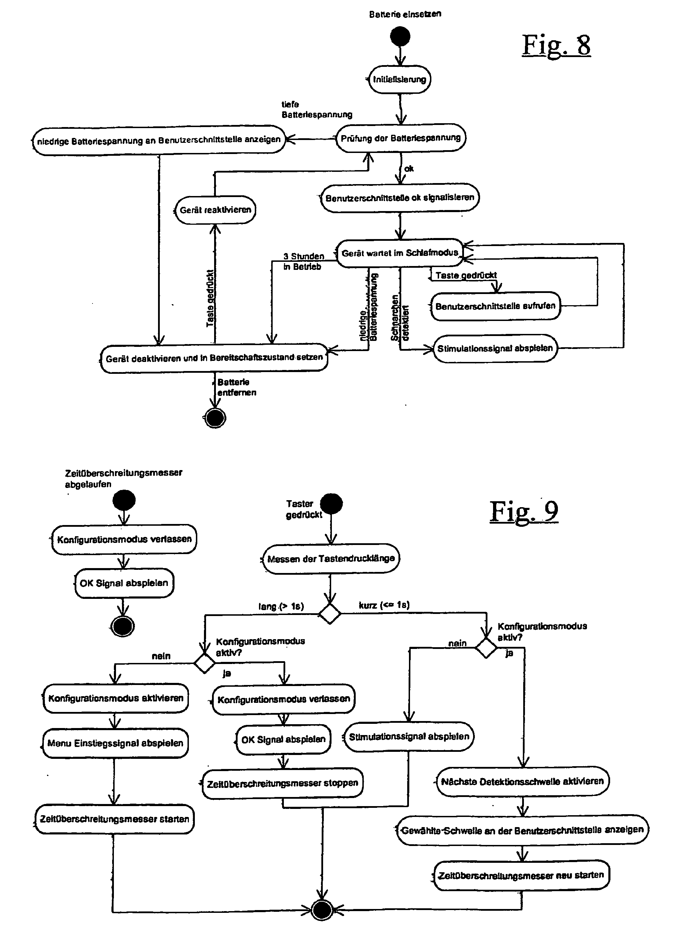

Fig. 8 ist ein Schaubild der Funktionen des Geräts auf der obersten Ebene. Es geht hierbei um die Vorgänge nach dem Einschalten des Geräts zur Detektion von Schnarchgeräuschen.

Das Einschalten erfolgt z. B. mit dem Stellknopf 32 am Potentiometer 30 oder alternativ durch Einsetzen einer zwischenzeitlich herausgenommenen Batterie 24 in das unter der Verschlussklappe 36 liegende Batteriefach. Das Einschalten ist in Fig. 8 als Initialisierung bezeichnet. Darauf folgt als erstes eine Prüfung der Batteriespannung. Ist sie zu niedrig, also die Batterie weitgehend erschöpft, erfolgt eine Meldung über die Benutzerschnittstelle, d. h. der digitale Signalprozessor 20 löst über den elektroakustischen Wandler 28 ein bestimmtes Anzeigesignal, z. B. drei Piepstöne, aus. Danach geht das Gerät in den Bereitschaftszustand, in dem es nicht in der Lage ist, Schnarchen zu detektieren. Wenn der Benutzer das wegen weitgehend entladener Batterie deaktivierte Gerät durch Drücken des Tasters 34 zu reaktivieren versucht, wiederholt sich der vorstehend beschriebene Vorgang.

Werden im Schlafmodus Schnarchgeräusche registriert, z. B. mindestens drei Schnarchgeräusche mit Intervallen von 3 bis 8 Sekunden und mit einer im Frequenzbereich von 10 bis 100 Hz über einem bestimmten Ansprechniveau liegenden Energie, erfolgt nach entsprechender Signalgebung des elektroakustischen Wandlers 28 in seiner Mikrofonfunktion an den Signalprozessor 20 von dort ein Steuerbefehl zum Auslösen eines Stimulationssignals durch den elektroakustischen Wandler 28. Danach wartet das Gerät, ob eine weitere Folge von drei Schnarchgeräuschen folgt. Wenn dies der Fall ist, wird ein weiteres Stimulationssignal durch den elektroakustischen Wandler 28 erzeugt, dieses jedoch nicht vor Ablauf einer bestimmten Zeitdauer von z. B. 15 Sekunden nach dem vorangehenden Stimulationssignal. Der erzeugte Schall kann z. B. die Form einer Sinusschwingung mit etwa 600 Hz haben.

Wenn während des Betriebs des Geräts im Schlafmodus die Batteriespannung unter einen bestimmten Grenzwert fällt, wird der Schlafmodus ausgeschaltet, und das Gerät geht in den Bereitschaftszustand über, ohne dass dies vom elektroakustischen Wandler 28 angezeigt wird. Selbst wenn die Batterieladung noch für eine längere Betriebsdauer im Schlafmodus ausreichen würde, schaltet das Gerät nach drei Stunden selbsttätig vom Schlafmodus in den Bereitschaftsmodus zurück. In dieser Zeit sollte eine normalerweise durch das Schnarchen gestörte Person infolge der mittels des Geräts verhinderten Schnarchgeräusche Gelegenheit gefunden haben, einzuschlafen. Außerdem vermeidet man weitgehend eine Störung der REM-Phase im Schlaf des Benutzers. Schließlich wird durch das selbsttätige Umschalten auf den Bereitschaftsmodus elektrische Energie gespart, so dass das Gerät mit einer kleineren Batterie auskommt.

Falls gewünscht, kann während des Schlafmodus durch Drücken des Tasters 34 die Benutzerschnittstelle aufgerufen werden, um z. B. die Empfindlichkeit des elektroakustischen Wandlers in der Mikrofonfunktion zu verändern. Danach erfolgt eine Rückkehr in den Schlafmodus zur weiteren Detektierung von Schnarchgeräuschen.

Die Funktionen der erwähnten Benutzerschnittstelle sind in Fig. 9 dargestellt. Sie kann nach der Initialisierung und Anzeige des OK-Signals durch Druck auf den Taster 34 angesprochen werden, und zwar wahlweise durch einen kurzen Druck von z. B. weniger als 1 Sekunde oder durch einen länger als 1 Sekunde währenden Druck. Da sich das Gerät bisher noch nicht im Konfigurationsmodus befindet, bewirkt ein kurzer Druck auf den Taster 34 nur ein Abfragen der Stellung des Potentiometers und die Anzeige, d. h. das Abspielen des Stimulationssignals mit der eingestellten Stärke durch den elektroakustischen Wandler 28. Das Gerät bleibt danach im Schlafmodus.

Durch einen länger als 1 Sekunde währenden Druck auf den Taster 34 wird der Konfigurationsmodus des Geräts aktiviert, was durch Abspielen eines akustischen Signals angezeigt wird, das die eingestellte Empfindlichkeitsstufe der Mikrofonfunktion des elektroakustischen Wandlers 28 repräsentiert. So können z. B. ein, zwei oder drei Piepstöne die erste, zweite bzw. dritte Empfindlichkeitsstufe anzeigen. Mit jedem weiteren kurzen Druck auf den Taster 34 wird jeweils die nächste Empfindlichkeitsstufe eingeschaltet und akustisch durch die jeweils zugeordneten Piepstöne angezeigt. Nach Erreichen der höchsten Empfindlichkeitsstufe und einem weiteren kurzen Druck auf den Taster 34 springt das Gerät in die erste Empfindlichkeitsstufe zurück, so dass man, ausgehend von einer zu Beginn noch eingestellt gewesenen Empfindlichkeitsstufe und deren Anzeige durch ein- oder mehrfaches kurzes Drücken des Tasters 34 in jede andere der vorgesehenen z. B. drei oder mehr Empfindlichkeitsstufen gelangen kann.

Wenn nach Einstellung und akustischer Anzeige einer bestimmten Empfindlichkeitsstufe während einer bestimmten Zeitdauer von z. B. 3 Sekunden kein weiterer Druck auf den Taster 34 erfolgt, wird der Konfigurationsmodus automatisch beendet und das Gerät geht wieder in den Schlafmodus zurück. Es kann vorgesehen sein, dass dieselbe Wirkung auch dann eintritt, wenn man nach Einschaltung des Konfigurationsmodus länger als 1 Sekunde auf den Taster 34 drückt. Dann verlässt man sofort den Konfigurationsmodus, und es wird das OK-Signal abgespielt, um anzuzeigen, dass man sich wieder im Schlafmodus mit Detektion von Schnarchgeräuschen befindet.

## Patentansprüche

1. Antischnarchgerät mit einem Tragorgan (10), an dem ein Mikrofon und ein Signalprozessor (20) zur Detektion von Schnarchgeräuschen, ein an ein Ohr anzusetzender Tongenerator zur Erzeugung eines akustischen Stimulationssignals und ein elektrischer Energiespeicher (24) angebracht sind, wobei das Mikrofon und der Tongenerator durch einen einzigen elektroakustischen Wandler (28) gebildet sind, das gemeinsame Tragorgan ein im Ohr zu tragendes Gehäuse (10) ist und die Detektion nach einer bestimmten Zeitdauer selbsttätig ausschaltbar ist,
**dadurch gekennzeichnet, dass**
der Signalprozessor (20) nur dann ein Stimulationssignal auslöst, wenn die Schallenergie im 10- bis 100-Hz-Frequenzbereich ein bestimmtes Ansprechniveau überschreitet und mindestens drei Schnarthgeräusche mit einem Intervall von 3 bis 8 Sekunden registriert wurden.

2. Antischnarchgerät nach Anspruch 1, **dadurch gekennzeichnet, dass** das Gehäuse (10) mit Steuerorganen (32, 34) zur Einstellung der Lautstärke des Stimulationssignals und der Aufnahme-Empfindlichkeit des elektroakustischen Wandlers (28) versehen ist und durch diesen auch die Einstellung der Lautstärke des Stimulationssignals und der Aufnahmeempfindlichkeit sowie der Ladezustand des elektrischen Energiespeichers (24) anzeigbar sind.

3. Antischnarchgerät nach Anspruch 1 oder 2, **dadurch gekennzeichnet, dass** das Gehäuse (10) aus einem Unterteil (11) und einem mit diesem verbundenen Deckel (12) besteht, wobei das Steuerorgan für die Lautstärke des Stimulationssignals ein im Gehäuse (10) installiertes Potentiometer (30) mit einem auf der Außenseite des Deckels (12) angebrachten Stellknopf (32) und das Steuerorgan für die Einstellung der Aufnahme-Empfindlichkeit ein mit dem Signalprozessor (20) zusammenwirkender, auf der Außenseite des Deckels zugänglicher Taster (34) ist.

4. Antischnarchgerät nach Anspruch 3, **dadurch gekennzeichnet, dass** der Stellknopf (32) des Potentiometers (30) auch der Ein- und Ausschalter des Geräts ist.

5. Antischnarchgerät nach einem der Ansprüche 1 bis 4, **dadurch gekennzeichnet, dass** die äußere Form des Gehäuses (10) individuell der Innenkontur eines Gehörgangs angepasst ist.

6. Antischnarchgerät nach einem der Ansprüche 1 bis 4, **dadurch gekennzeichnet, dass** das den Tonein- und -ausgang (14) für den elektroakustischen Wandler (28) bildende untere Ende des Gehäuses (10) mit einem Anschluss (15) für unterschiedlich große Paßstücke (16) versehen ist, die den Tonein- und -ausgang (14) verlängern.

7. Antischnarchgerät nach Anspruch 3 oder 4, **dadurch gekennzeichnet, dass** das Potentiometer (30), der Signalprozessor (20) und Kontakte (22) für eine Batterie (24) oder einen Akkumulator an der Innenwand des Deckels (12) befestigt sind.

8. Antischnarchgerät nach einem der vorhergehenden Ansprüche, **dadurch gekennzeichnet, dass** die Detektion nach zwei bis drei Stunden selbsttätig ausschaltbar ist.

9. Antischnarchgerät nach einem der vorhergehenden Ansprüche, **dadurch gekennzeichnet, dass** das Intervall zwischen zwei Stimulationssignalen mindestens 15 Sekunden dauert.

## Claims

1. An anti snore device comprising a wearable element (10) on which there are mounted a microphone and a signal processor (20) for the detection of snoring noises, a tone generator to be fitted to an ear for the generation of an acoustic stimulation signal, and an electrical energy storage device (24), wherein said microphone and said tone generator are formed by a single electroacoustic transducer (28), said basic wearable element is a housing (10) adapted to be worn in an ear, and said detection means are adapted to be automatically switched off following a specified period of time,
**characterized in that**
said signal processor (20) triggers a stimulation signal only when the sound energy in the 10 Hz to 100 Hz frequency range exceeds a specified response level and when at least three snoring noises have been detected at intervals of from 3 to 8 seconds.

2. The anti snore device according to claim 1, **characterized in that** said housing (10) is provided with control elements (32, 34) for adjusting the volume of said stimulation signal and the detection sensitivity of said electroacoustic transducer (28), by which means the volume setting of said stimulation signal and the detection sensitivity level of said electroacoustic transducer (28), and also the charge level of said electrical energy storage device (24) are additionally displayable.

3. The anti snore device according to claim 1 or claim 2, **characterized in that** said housing (10) consists of a base portion (11) and a covering member (12) connected thereto, wherein said control element for adjusting the volume of said stimulation signal is a potentiometer (30) installed in said housing (10) and having an adjustment knob (32) mounted on the external surface of said covering member (12) and said control element for setting the detection sensitivity of said electroacoustic transducer is a pushbutton (34) that is adapted to cooperate with said signal processor (20) and is accessible on the external surface of said covering member.

4. The anti snore device according to claim 3, **characterized in that** said adjustment knob (32) for said potentiometer (30) is also the on/off switch of said device.

5. The anti snore device according to any one of claims 1 to 4, **characterized in that** the exterior shape of said housing (10) is customized to match the interior contour of an ear canal.

6. The anti snore device according to any one of claims 1 to 4, **characterized in that** the lower end of said housing (10) forming the audio input/output (14) for said electroacoustic transducer (28) is provided with connection means (15) for accommodating differently dimensioned adapters (16) for extending said audio input/output (14).

7. The anti snore device according to claim 3 or claim 4, **characterized in that** said potentiometer (30), said signal processor (20), and contacts (22) for a battery or accumulator (24) are mounted on the inside wall of said covering member (12).

8. The anti snore device according to any one of the preceding claims, **characterized in that** said detection means are adapted to be automatically switched off after from two to three hours.

9. The anti snore device according to any one of the preceding claims, **characterized in that** the interval between any two stimulation signals is at least 15 seconds.

## Revendications

1. Appareil anti-ronflement, avec un organe porteur (10) sur lequel sont placés un microphone et un processeur de signaux (20) pour la détection de bruits de ronflement, un générateur de sons à appliquer contre une oreille pour produire un signal de stimulation acoustique et un accumulateur d'énergie électrique (24), sachant que le microphone et le générateur de sons sont formés par un unique convertisseur électroacoustique (28), que l'organe porteur commun est un boîtier (10) à porter dans l'oreille et que la détection est automatiquement désactivable au bout d'une durée déterminée,
**caractérisé en ce que** le processeur de signaux (20) ne déclenche un signal de stimulation que si l'énergie acoustique dépasse un niveau de réponse déterminé dans la plage de fréquences de 10 à 100 Hz et si au moins trois bruits de ronflement avec un intervalle de 3 à 8 secondes ont été enregistrés.

2. Appareil anti-ronflement selon la revendication 1, **caractérisé en ce que** le boîtier (10) est pourvu d'organes de commande (32, 34) pour régler le volume sonore du signal de stimulation et la sensibilité d'enregistrement du convertisseur électroacoustique (28), et ces organes permettent également d'afficher le réglage du volume sonore du signal de stimulation et la sensibilité d'enregistrement, ainsi que l'état de charge de l'accumulateur d'énergie électrique (24).

3. Appareil anti-ronflement selon la revendication 1 ou 2, **caractérisé en ce que** le boîtier (10) est constitué dune partie inférieure (11) et d'un couvercle (12) assemblé à celle-ci, sachant que l'organe de commande pour le volume sonore du signal de stimulation est un potentiomètre (30) installé dans le boîtier (10) et doté d'un bouton de réglage (32) placé sur le côté extérieur du couvercle (12), et que l'organe de commande pour le réglage de la sensibilité d'enregistrement est un bouton poussoir (34) coopérant avec le processeur de signaux (20) et accessible sur le côté extérieur du couvercle.

4. Appareil anti-ronflement selon la revendication 3, **caractérisé en ce que** le bouton de réglage (32) du potentiomètre (30) est également le commutateur marche/arrêt de l'appareil.

5. Appareil anti-ronflement selon l'une des revendications 1 à 4, **caractérisé en ce que** la forme extérieure du boîtier (10) est individuellement adaptée au contour intérieur d'un conduit auditif.

6. Appareil anti-ronflement selon l'une des revendications 1 à 4, **caractérisé en ce que** l'extrémité inférieure du boîtier (10), qui forme l'entrée/sortie de sons (14) pour le convertisseur électroacoustique (28), est pourvue d'un raccord (15) pour des adaptateurs (16) de différentes tailles qui prolongent l'entrée/sortie de sons (14).

7. Appareil anti-ronflement selon la revendication 3 ou 4, **caractérisé en ce que** le potentiomètre (30), le processeur de signaux (20) et des contacts (22) pour une batterie (24) ou un accumulateur sont fixés sur la paroi intérieure du couvercle (12).

8. Appareil anti-ronflement selon l'une des revendications précédentes, **caractérisé en ce que** la détection peut être désactivée automatiquement au bout de deux à trois secondes.

9. Appareil anti-ronflement selon l'une des revendications précédentes, **caractérisé en ce que** l'intervalle entre deux signaux de stimulation dure au moins 15 secondes.
